# EUROPÄISCHE PATENTSCHRIFT

(11) **EP 0 745 352 B1**
(45) Veröffentlichungstag und Bekanntmachung des Hinweises auf die Patenterteilung: **29.08.2001**
(21) Anmeldenummer: 96107809.4
(22) Anmeldetag: 15.05.1996
(51) Int. Cl.: A61B 17/16

(54) **Knochenmarkraspel**
Intramedullary rasp
Râpe intramédullaire

(30) Priorität: 31.05.1995 DE 19519971
(43) Veröffentlichungstag der Anmeldung: 04.12.1996
(73) Patentinhaber: Szabo, Zsolt, D-80933 München (DE)
(72) Erfinder: Szabo, Zsolt, D-80933 München (DE)
(74) Vertreter: Becker Kurig Straus

(56) Entgegenhaltungen:
- EP-A- 0 296 986
- EP-A- 0 634 145
- DE-C- 4 116 507
- DE-U- 9 212 906
- FR-A- 2 547 192

## Beschreibung

Vorliegende Erfindung betrifft eine Knochenmarkraspel zum Bilden von Knochenhohlräumen gemäß dem Oberbegriff des Anspruchs 1. Eine derartige Knochenmarkraspel ist bsp. aus der FR-A-2547192 bekannt.

Aus der DE 39 07 256 A1 ist bereits eine Knochenraspel zur Schaffung eines Schaftbettes für eine Hüftgelenks-Endoprothese im Hohlraum des menschlichen Oberschenkelknochens bekannt, die einen länglichen Hohlkörper aufweist, der einen innenliegenden Abführkanal aufweist. Dieser Abführkanal ist durch Verbindungsbohrungen mit der Außenfläche des Körpers verbunden. Ferner sind längs des Umfanges des Körpers verteilte Schneidelemente vorgesehen. Der Hauptkörper ist an seinem distalen Ende geschlossen.

Der menschliche Oberschenkelknochen hat eine harte Außenhaut und eine innenliegende schwammartige Masse, die man als Spongiosa bezeichnet. Diese hochviskose-schwammartige Masse muß entfernt werden, um für die einzusetzende Prothese Platz zu schaffen. Die Prothese wird in den gebildeten Knochenhohlraum eingesetzt, und kann auch mit Hilfe von Knochenzement befestigt werden.

Zur Vorbereitung und Bildung des gewünschten Knochenbettes sind früher Vollraspeln eingesetzt worden, welche die Form der einzusetzenden Prothesen haben und in aufsteigenden Größen nacheinander axial in den Knochen eingeschlagen werden. Derartige Vollraspeln sind aus massivem Material gebildet und drücken die in der Spongiosa vorhandene Flüssigkeit nach außen. Dadurch besteht zum einen die Gefahr, den Knochen aufgrund des radialen Druckes zu sprengen. Zum anderen besteht die Gefahr, daß in der Flüssigkeit enthaltene Mikroteilchen und Fettpartikel in die Blutbahn gelangen und beispielsweise in der Lunge Embolien verursachen.

Dieses sog. Fettembolierisiko ist bei der Implantation von zementierten Hüftgelenksprothesen bzw. Hüftendoprothesen in der Orthopädie seit 1970 bekannt.

Nachdem auch bei Operationen mit zementfreien Prothesen immer wieder Komplikationen, teilweise sogar mit Todesfolge, auftraten, begann eine intensive Suche nach den Ursachen. Nach dem heutigen Stand der Wissenschaft entstehen durch das Einschlagen der herkömmlichen Vollraspel koaxiale und radiale Druckbelastungen, die intraossäre Druckspitzen zur Folge haben. Es kommt zur Ausschüttung von Knochenmark in die venöse Blutbahn. Die Knochenmarksbestandteile aktivieren das Blutgerinnungssystem, wodurch es im Zusammenhang mit der interoperativ ohnehin reduzierten Blutfließgeschwindigkeit zur Bildung von Macroemboli kommt. Diese Emboli bzw. Thromben gelangen über die Blutbahn in die Lunge und führen zu einer Lungenembolie, einem potentiell lebensgefährlichen Krankheitsbild. Dieses Krankheitsbild ist unter dem Begriff Fettemboliesyndrom (FES) bekannt.

Die aus der obengenannten DE 39 07 256 A1 bekannt Knochenraspel leistet einen ersten Schritt zur Lösung des Problems, da durch die seitlich mündenden Bohrungen Knochenmaterial abgeführt werden kann. Die Wirkung dieser Knochenraspel ist jedoch unzureichend, da sie nur geringe Unterschiede zur Vollraspel aufweist und nur einen Teil des seitlich auftreffenden Knochenmaterials abführt.

In "Der Orthopäde", Band 24, Hf 2, April 1995, wird in einer Vielzahl von unterschiedlichen Beiträgen von auf diesem Gebiet tätigen Fachleuten auf den direkten Zusammenhang zwischen pulmonalen Fettembolien und Hüftgelenks-Operationen in Folge intramedulärer Druckerhöhung hingewiesen.

Aufgabe der vorliegenden Erfindung besteht in der Angabe einer Knochenmarkraspel, bei deren Anwendung die pathogene Wirkung auf den Patienten vermindert wird. Insbesondere ist Aufgabe der Erfindung, den lateralen Druck bei der Einführung der Raspel in den Knochen zu vermindern und das gelöste Knochenmaterial wirksam abzuführen. Schließlich sollen die Raspelelemente stabilisiert und gegen Verformung geschützt werden.

Durch Reduzierung der Vorschubkraft beim Raspeln und Bohren können die intramedullären Druckspitzen verringert und die Knochenmarksausschüttung gesenkt werden. Dabei ist darauf zu achten, daß die Verdrängung des Knochenmarks reduziert wird und dadurch ein Druckaufbau im Knocheninneren vermieden wird.

Erfindungsgemäß wird angegeben eine Knochenmarkraspel gemäß Anspruch 1.

Die erfindungsgemäße Knochenmarkraspel verhindert einen axialen und radialen Überdruck und ermöglicht eine schonende Markraumvorbereitung bei der Implantation von Endoprothesen-Schäften. In Vorschubrichtung ist die Raspel durch eine speziell definierte Geometrie für einen Materialdurchtritt bis zu 90 Prozent geöffnet. Damit wird die seitliche Verdrängung von Knochenmarkvolumen erheblich reduziert. Knochenflüssigkeit wird vornehmlich in das Innere der Raspel geleitet, so daß der gewünschte Verdichtungseffekt der Spongiosa bestehen bleiben kann. Das Gewicht der erfindungsgemäßen Raspel ist aufgrund der Öffnungen gering.

Die erfindungsgemäße Knochenmarkraspel stellt einen an seinem distalen Ende geöffneten rohrartigen Hohlkörper dar, der somit ohne wesentliche radiale Verdrängung in die Spongiosa bzw. das Knochenmark eingeführt werden kann. Dies geschieht mit geringem Kraftaufwand. Das axial stirnseitig auf den Hohlkörper auftreffende Knochenmaterial wird entlang des im Hohlkörper bestehenden Hohlraumes abgeführt. Die Raspel baut bei der Einführung minimalen Druck im Knochen auf. Andererseits wird das durch die Raspelelemente lateral abgelöste Knochenmaterial unmittelbar in den Raspelelementen benachbarte Öffnungen geführt, die mit dem Hohlraum in Verbindung stehen und das Knochenmaterial wirksam und schnell abführen. Da der Hohlkörper eine im Vergleich zu seinem Durchmesser geringe Wandstärke aufweist, erreicht das durch die Öffnungen geführte Knochenmaterial den Hohlraum schnell und kann wirksam axial abgeführt werden.

Jedes Raspelelement wird durch eine in sich geschlossene radial umlaufende Schneide gebildet, mit der in axialer Richtung davor eine entsprechende radial umlaufende Öffnung korrespondiert, in die das von dem Raspelelement abgehobene Knochenmaterial ein- bzw. abgeführt wird. Somit ist die effektive Raspelfläche gegenüber einzelnen räumlich begrenzten Raspelelementen deutlich vergrößert, und die Knochenmarkabhebung wirksam gesteigert.

Die Raspelelemente sind in einer bevorzugten Ausführungsform durch eine einzige spiralförmig umlaufende Schneide gebildet. Im Gegensatz zu der vorangegangenen Ausführungsform ist somit eine einzelne helixförmig umlaufende Schneide gebildet, welche die erfindungsgemäßen Raspelelemente definiert. Diese spiralförmige Schneide korrespondiert mit einer entsprechend spiralförmigen Öffnung in axialer Richtung vor der Schneide.

Die umlaufenden Raspelelemente sind durch mindestens einen Verbindungssteg in axialer Richtung verbunden. Der Verbindungssteg stützt die in der Wand des Hohlkörpers gebildeten umlaufenden Raspelelemente wirksam ab, so daß sie sich bei der Einführung in den Knochen nicht verformen.

Weitere Vorteile, Merkmale und Anwendungsmöglichkeiten der vorliegenden Erfindung ergeben sich aus der nachfolgenden Beschreibung zweier Ausführungsbeispiele in Verbindung mit der Zeichnung.
- Figur 1: zeigt eine Seitenansicht einer ersten Ausführungsform einer Knochenmarkraspel gemäß der Erfindung mit spiralförmiger Schneide.
- Figur 2: zeigt die Raspel von Figur 1 in perspektivischer Ansicht von vorne.
- Figur 3: zeigt eine weitere Ausführungsform einer Raspel gemäß der Erfindung in perspektivischer Ansicht.
- Figur 4: zeigt die Raspel von Figur 3 von vorne in perspektivischer Ansicht.
- Figur 5: zeigt die Raspel von Figur 3 im Schnitt.

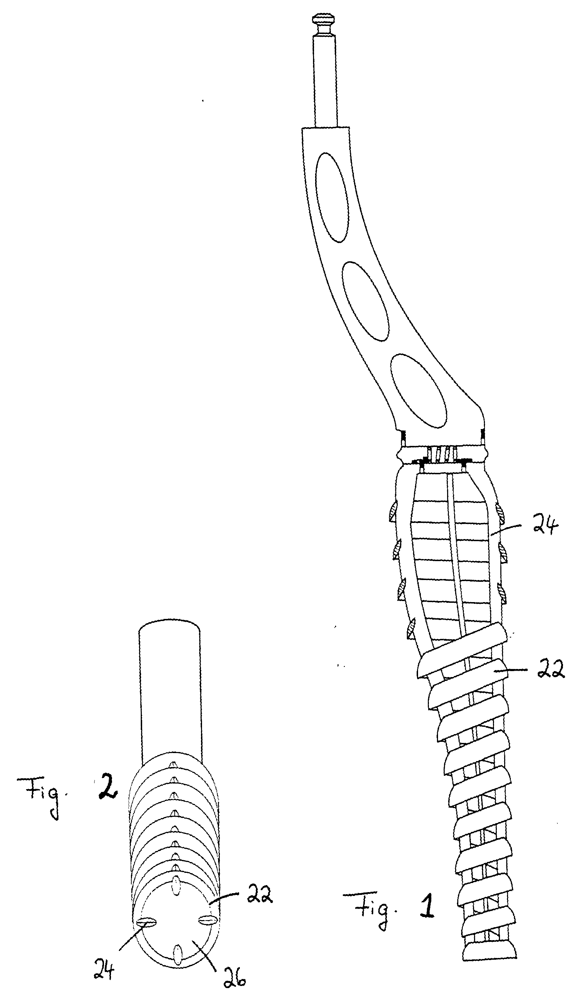

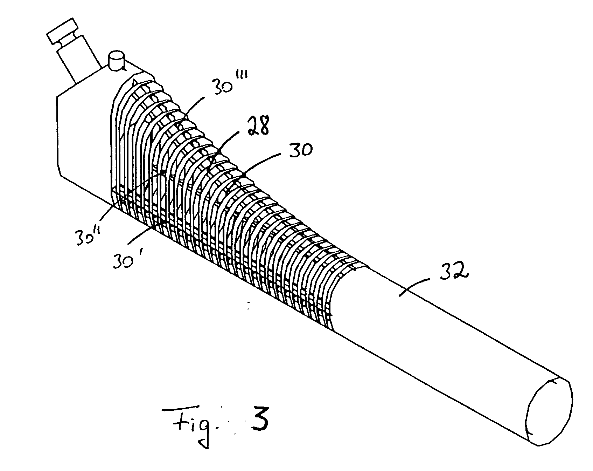

Figur 1 und 2 zeigen eine erste Ausführungsform einer Knochenmarkhohlraspel, wobei die Raspelelemente durch eine spiralförmig gewundene Schneide 22 gebildet sind, welche durch Seitenstege 24 stabilisiert ist. Es sind vier in 90 Grad gegeneinander versetzte Seitenstege 24 vorgesehen, die axial stirnseitig durch einen Abschlußring 26 abgeschlossen sind.

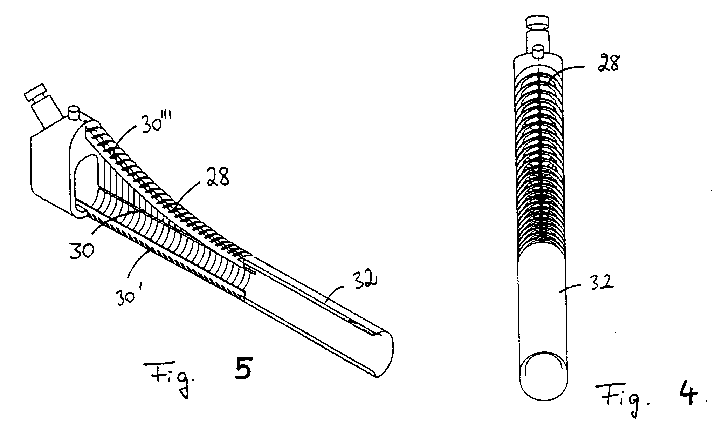

Figuren 3, 4 und 5 zeigen eine weitere Ausführungsform einer Knochenmarkraspel mit umlaufenden spiralförmigen bzw. helixförmigen Raspelelementen, die durch eine einzige helixförmige Schneide 28 gebildet sind. Durch die radial spiralförmig umlaufende Schneide 28 sind lamellenartige Raspelelemente gebildet, deren Schneiden sich im wesentlichen in axialer Richtung erstrecken und wirksam zur Abhebung von Knochenmaterial dienen. Die Schneide 28 ist in der Wand des Hohlkörpers gebildet bzw. Teil dieser Wand, so daß eine mit der Schneide 28 kooperierende Öffnung ebenfalls spiralförmig umläuft. In Figur 3 sind vier Verbindungsstege 30, 30', 30", 30''' gebildet, welche die Raspelelemente in axialer Richtung durchsetzen bzw. verbinden, um deren Stabilität und Steifigkeit zu erhöhen. Stirnseitig wird die Hohlraspel abgeschlossen durch ein tubusförmiges, nicht verjüngtes Rohrelement 32, welches die Hohlraspel stirnseitig abschließt und keine Raspelelemente enthält.

Die Ausführungsformen nach Figuren 1 bis 5 werden bevorzugt hergestellt im Hochdruckguß aus einem medizinisch verträglichen Material.

## Patentansprüche

1. Knochenmarkraspel zum Bilden von Knochenhohlräumen, insbesondere im Oberschenkelknochen mit einem rohrartigen Hohlkörper, der an seinem distalen Ende zur axial gerichteten Aufnahme von Knochenmaterial offen ist, längs seines Umfangs gebildete Öffnungen aufweist, die mit dem Hohlraum zur Abführung von Knochenmaterial in Verbindung stehen, und entlang seiner Außenfläche mit Raspelelementen versehen ist, die mit den Öffnungen zusammenwirken, wobei die Raspelelemente lamellenartig ausgebildet sind und eine radial umlaufende Kontur haben, **dadurch gekennzeichnet, daß** die Raspelelemente durch mindestens einen Verbindungssteg (24, 30, 30', 30", 30''') stabilisiert sind, der die Raspelelemente (22, 28) in axialer Richtung verbindet, wobei die in axialer Richtung vor den Raspelelementen vorgesehenen Öffnungen ebenfalls radial umlaufen

2. Knochenmarkraspel nach Anspruch 1, **dadurch gekennzeichnet, daß** die Raspelelemente durch eine einzige spiralförmig umlaufende Schneide (22, 28) gebildet sind.

## Claims

1. Intramedullary rasp for forming bone cavities, more especially in the femur, said rasp having a tubular hollow body, which is open at its distal end for the axially orientated accommodation of bone material and has openings, which are formed along its circumference and communicate with the cavity for the removal of bone material, and said hollow body is provided, along its external surface, with rasp elements which co-operate with the openings, the rasp elements having a lamellar configuration and a radially extending contour, **characterised in that** the rasp elements are stabilised by at least one connecting web (24, 30, 30', 30", 30"'), which connects the rasp elements (22, 28) in the axial direction, the openings provided in front of the rasp elements, when viewed with respect to the axial direction, also extending radially.

2. Intramedullary rasp according to claim 1, **characterised in that** the rasp elements are formed by a single, helically extending cutting edge (22, 28).

## Revendications

1. Râpe intramédullaire pour former des cavités osseuses, notamment dans l'os de la cuisse, comportant un corps creux tubulaire, qui est ouvert à son extrémité distale afin de recueillir en direction axiale de la matière osseuse, qui comporte des ouvertures, formées le long de sa périphérie et en liaison avec la cavité pour évacuer la matière osseuse, et qui est muni le long de sa surface extérieure d'éléments de râpe, qui coopèrent avec les ouvertures, qui sont conçus comme des lamelles et qui ont un contour radialement périphérique, **caractérisée en ce que** les éléments de râpe sont stabilisés par au moins une traverse de liaison (24, 30, 30', 30'', 30''') qui relie les éléments de râpe (22, 28) en direction axiale, les ouvertures prévues en direction axiale devant les éléments de râpe étant également radialement périphériques.

2. Râpe intramédullaire selon la revendication 1, **caractérisée en ce que** les éléments de râpe sont formés par un seul tranchant (22, 28) périphérique en forme de spirale.
